(19) Europäisches Patentamt
European Patent Office
Office européen des brevets

(11) Veröffentlichungsnummer: **0 268 797 A2**

(12) **EUROPÄISCHE PATENTANMELDUNG**

(21) Anmeldenummer: **87114775.7**

(22) Anmeldetag: **09.10.87**

(51) Int. Cl.4: **C07D 209/48**, C07D 471/04, C07D 231/56, C07D 271/10, C07D 249/12, A01N 43/38, A01N 43/56, A01N 43/82, A01N 43/88, A01N 43/90

(30) Priorität: **14.10.86 DE 3635309**

(43) Veröffentlichungstag der Anmeldung: **01.06.88 Patentblatt 88/22**

(84) Benannte Vertragsstaaten: **AT BE CH DE ES FR GB GR IT LI LU NL SE**

(71) Anmelder: **SCHERING AKTIENGESELLSCHAFT**
**Berlin und Bergkamen**
**Müllerstrasse 170/178 Postfach 65 03 11**
**D-1000 Berlin 65(DE)**

(72) Erfinder: **Blume, Friedhelm, Dr.**
**Nusshäherstrasse 47 T**
**D-1000 Berlin 27(DE)**
Erfinder: **Franke, Wilfried, Dr.**
**Karl-Stieler-Strasse 2 b**
**D-1000 Berlin 41(DE)**
Erfinder: **Arndt, Friedrich, Dr.**
**Mühlenfeldstrasse 10**
**D-1000 Berlin 28(DE)**
Erfinder: **Rees, Richard, Dr.**
**Speerweg 8**
**D-1000 Berlin 28(DE)**

(54) **Halogencyclopropyl-Verbindungen, ihre Herstellung und Verwendung als herbizide Mittel.**

(57) Es werden neue Halogencyclopropyl-Verbindungen der allgemeinen Formel I

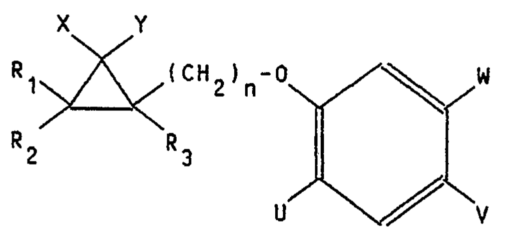

(I) ,

worin
$R_1$, $R_2$ und $R_3$ unabhängig voneinander Wasserstoff oder $C_{1-4}$-Alkyl,
X Wasserstoff oder Halogen,
Y Halogen
n 0, 1, 2 oder 3
U und V Wasserstoff oder Halogen und
W einen heterocyclischen Rest der Formeln

EP 0 268 797 A2

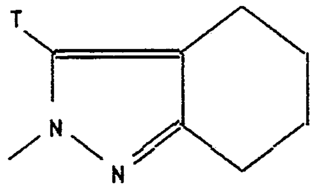 ,

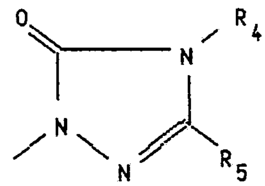 ,

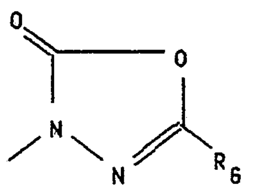 ,

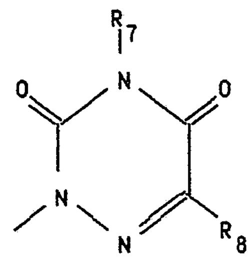 ,

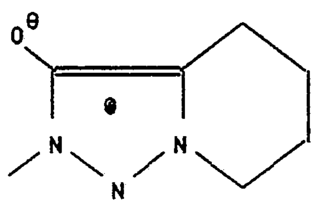 ,

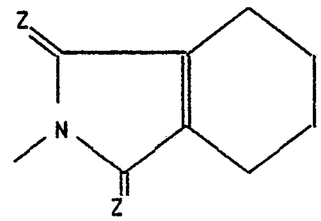

oder 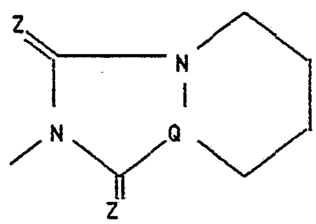

bedeuten, wobei
T für Halogen, $C_{1-4}$-Alkyl, $C_{1-4}$-Halogenalkyl, CN oder $OR_9$,
Q für CH oder N,
Z für O oder S,
$R_4$, $R_5$, $R_6$, $R_7$ und $R_8$ jeweils für einen geradkettigen, verzweigten oder cyclischen, gegebenenfalls mit bis zu sechs Halogenatomen substituierten $C_{1-7}$-Alkylrest stehen, wobei
$R_4$ und $R_5$ außerdem gemeinsam einen heterocylischen 4-bis 7-gliedrigen Ring bilden können, der gesättigt oder ungesättigt sein kann, weitere Heteroatome wie O, S oder N enthalten kann und gegebenenfalls mit ein bis drei Methylgruppen oder ein bis sechs Halogenatomen substituiert sein kann, und
$R_9$ Wasserstoff, C $_{1-4}$-Alkyl oder $C_{1-4}$-Halogenalkyl bedeutet, Verfahren zu ihrer Herstellung und ihre Verwendung als Herbizide mit hoher Selektivität beschrieben.

# HALOGENCYCLOPROPYL-VERBINDUNGEN, IHRE HERSTELLUNG UND VERWENDUNG ALS HERBIZIDE MITTEL

Die Erfindung betrifft neue Halogencyclopropyl-Verbindungen, ihre Herstellung nach an sich bekannten Methoden und ihre Verwendung als herbizide Mittel.

Es ist bereits bekannt, daß bestimmte Tetrahydroindazole und Tetrahydrophthalsäureimide herbizide Eigenschaften haven (EP 61 741 und 105 721). Die Wirksamkeit dieser Stoffe ist bei Vorauflauf-und Nachauflaufanwendung als gut zu bezeichnen. Nachteilig ist jedoch, daß ihre Verträglichkeit in einigen Kulturen - wie zum Beispiel bei Baumwolle, Soja und Getreide - nicht voll ausreicht.

Es wurde nun gefunden, daß Halogencyclopropyl-Verbindungen der allgemeinen Formel I

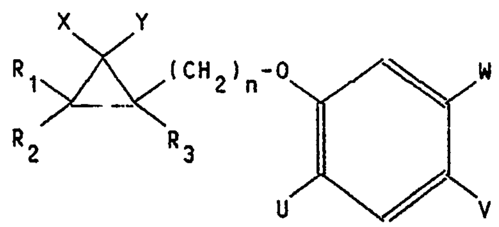

(I) ,

worin

$R_1$, $R_2$ und $R_3$ unabhängig voneinander wasserstoff oder $C_{1-4}$-Alkyl,
X Wasserstoff oder Halogen,
Y Halogen
n 0, 1, 2 oder 3
U und V Wasserstoff oder Halogen und
W einen heterocyclischen Rest der Formeln

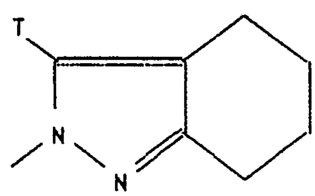 , 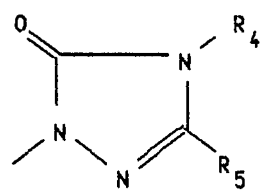 ,

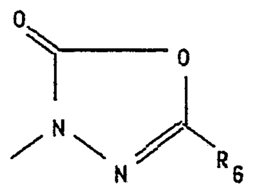 , 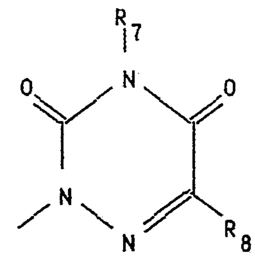 ,

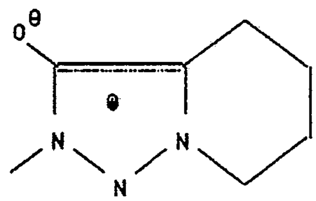 , 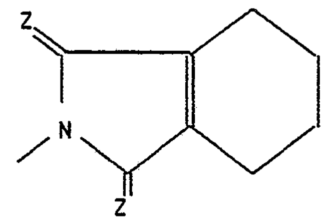

oder 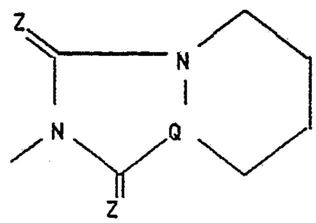 bedeuten,

wobei
T für Halogen, $C_{1-4}$-Alkyl, $C_{1-4}$-Halogenalkyl, CN oder $OR_9$,
Q für CH oder N,
Z für O oder S,
$R_4$, $R_5$, $R_6$, $R_7$ und $R_8$ jeweils für einen geradkettigen, verzweigten oder cyclischen, gegebenenfalls mit bis zu sechs Halogenatomen substituierten $C_{1-7}$-Alkylrest stehen, wobei
$R_4$ und $R_5$ außerdem gemeinsam einen heterocyclischen 4-bis 7-gliedrigen Ring bilden können, der gesättigt oder ungesättigt sein kann, weitere Heteroatome wie O, S oder N enthalten kann und gegebenenfalls mit ein bis drei Methylgruppen oder ein bis sechs Halogenatomen substituiert sein kann, und
$R_9$ Wasserstoff, $C_{1-4}$-Alkyl oder $C_{1-4}$-Halogenalkyl bedeutet,
überraschenderweise eine hervorragende Verträglichkeit für Reis, Weizen, Soja, Mais, Baumwolle und Gerste bei gleichzeitig verbesserter herbizider Wirkung zeigen.

Der Ausdruck Halogen steht für F, Cl, Br oder J. Die Bezeichnung Halogenalkyl besagt, daß ein oder mehrere Wasserstoffatome des Alkylrestes durch Halogen ersetzt sind.

Als Beispiele für heterocyclische Ringe seien genannt: Pyrrol, Oxazol, Thiazol, Imidazol, Pyridin, Oxazin, Thiazin, Pyrimidin, Pyrazin, Triazin, Oxadiazin und Thiadiazin sowie ihre Di-, Tetra-oder gegebenenfalls Hexahydroderivate.

Die erfindungsgemäßen Verbindungen können in an sich bekannter Weise auf folgenden Wegen dargestellt werden:

a) Ein Halogenid der allgemeinen Formel II

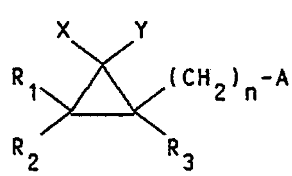 (II) ,

worin $R_1$, $R_2$, $R_3$, X, Y und n die in Formel I genannte Bedeutung haben A Chlor, Brom oder Jod bedeutet, wird mit einem Phenol der Formel III

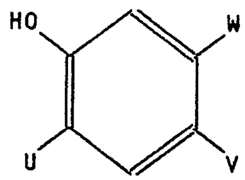

(III) ,

worin U, V und W die in der allgemeinen Formel I genannte Bedeutung haben, umgesetzt.

b) Zur Herstellung einer Verbindung der Formel I, in der W einen 4,5,6,7-Tetrahydroindazol-2-yl-Rest darstellt, wird ei ne Verbindung der Formel IV

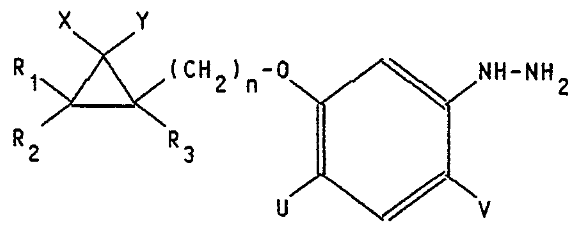

(IV) ,

worin $R_1$, $R_2$, $R_3$, X, Y, n, U und V die in Formel I genannte Bedeutung haben, mit einer Verbindung der Formel V

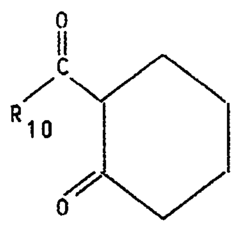

(V) ,

worin $R_{10}$ $C_{1-4}$-Alkyl, $C_{1-4}$-Halogenalkyl oder $C_{1-4}$ -Alkoxy bedeutet, umgesetzt.

Die so erhaltenen Verbindungen der Formel I mit T in der Bedeutung von Hydroxy können gegebenenfalls weiter umgesetzt werden. Zum Beispiel erhält man durch Umsetzung mit Dialkylsulfat die entsprechenden Verbindungen mit T in der Bedeutung von Alkoxy oder bei der Umsetzung mit Phosphoroxyhalogeniden erhält man die entsprecheden Verbindungen mit T in der Bedeutung von Halogen, die dann durch Umsetzung mit NaCN in die entsprechenden Verbindungen mit T in der Bedeutung von CN überführt werden können.

c) Zur Herstellung von Verbindungen der Formel I, in der W einen 2H-1,2,4-Triazolin-3-on-2-yl-Rest darstellt, wird eine Verbindung der Formel IV

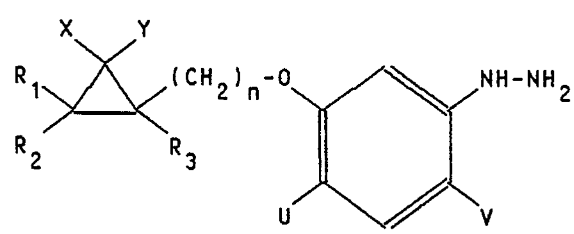

(IV) ,

worin $R_1$, $R_2$, $R_3$, X, Y, n, U und V die in Formel I genannte Bedeutung haben, mit einer Verbindung der Formel VI

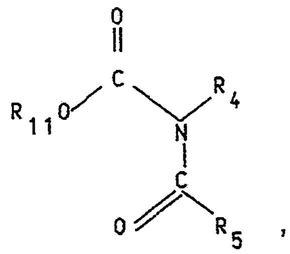

(VI) ,

worin $R_4$ und $R_5$ die in der Formel I genannte Bedeutung haben und $R_{11}$ $C_{1-4}$-Alkyl bedeutet, umgesetzt.

d) Zur Herstellung der Verbindungen der Formel I, in der W einen 3H-1,3,4-Oxadiazol-2-on-3-yl-Rest darstellt, wird eine Verbindung der Formel IV

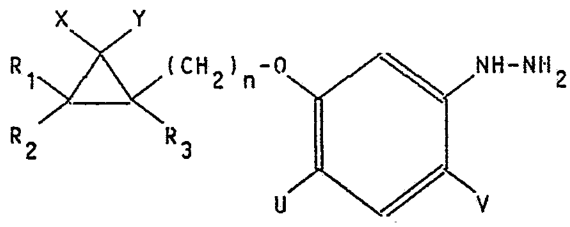

(IV) ,

worin $R_1$, $R_2$, $R_3$, X, Y, n, U und V die in Formel I genannte Bedeutung haben, mit einem Säurederivat der Formel VIII

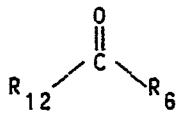

(VIII) ,

worin $R_6$ die in Formel I angegebene Bedeutung hat und $R_{12}$ Halogen oder C $_{1-4}$-Alkoxy bedeutet, intermediär zu einer Verbindung der Formel VII

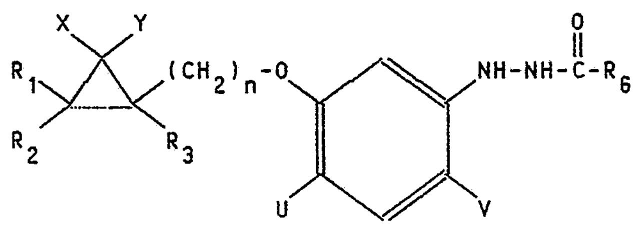

(VII) ,

in der $R_1$, $R_2$, $R_3$, X, Y, n, U, V und $R_6$ die in der Formel I genannte Bedeutung haben, umgesetzt und dann mit Phosgen oder einem seiner reaktiven funktionellen Derivate zur Reaktion gebracht.

e) Zur Herstellung von Verbindungen der Formel I, in der W einen 2H,4H-1,2,4-Triazin-3,5-dion-2-yl-Rest darstellt, wird eine Verbindung der Formel IV

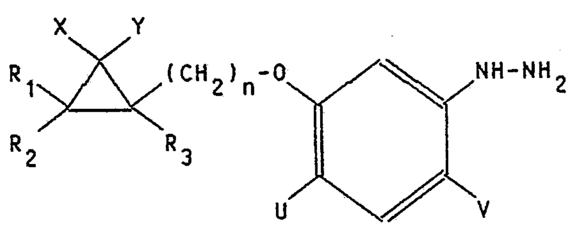

(IV) ,

worin $R_1$, $R_2$, $R_3$, X, Y, n, U und V die in Formel I genannte Bedeutung haben, mit einer Ketosäure der Formel XII

$R_8-\overset{O}{\overset{\|}{C}}-COOH$ (XII) ,

worin $R_8$ die in der Formel I genannte Bedeutung hat, zu einer Verbindung der Formel XI

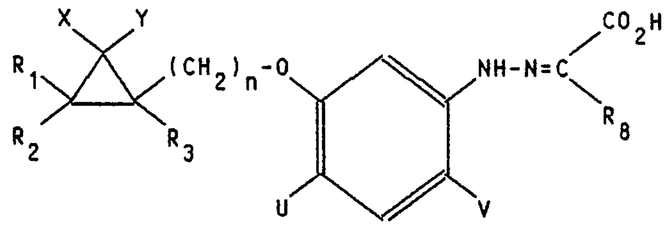

(XI) ,

worin $R_1$, $R_2$, $R_3$, X, Y, n, U, V und $R_8$ die in der Formel I genannte Bedeutung haben, umgesetzt, diese durch Behandlung mit Thionylchlorid oder Phosphoroxychlorid und anschließend mit Carbaminsäureestern cyclisiert und die so erhaltene Verbindung der Formel IX

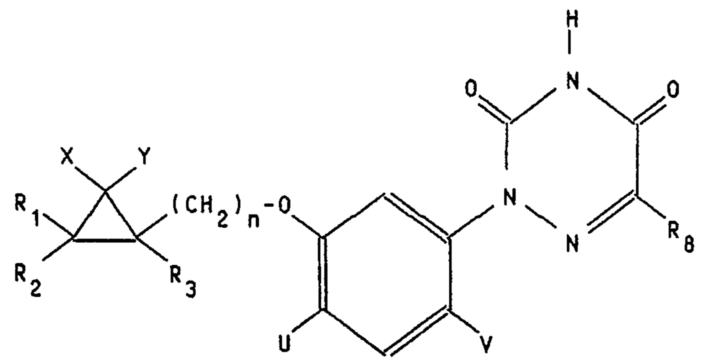

(IX) ,

worin $R_1$, $R_2$, $R_3$, X, Y, n, U, V und $R_8$ die in Formel I genannte Bedeutung haben, mit einer Verbindung der Formel X $R_7$-A (X) ,

worin $R_7$ die in Formel I genannte Bedeutung hat und A Chlor. Brom oder Jod bedeutet, umgesetzt.

f) Zur Herstellung von Verbindungen der Formel I, in der W einen 4,5,6,7-Tetrahydro-2H-1,2,3-triazolo[3,4-a]pyridin-8-ium-3-olat-2-yl-Rest darstellt, wird eine Verbindung der Formel XV

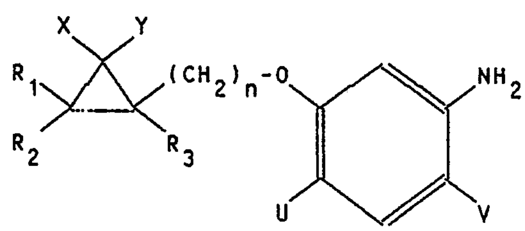

(XV) ,

worin $R_1$, $R_2$, $R_3$, X, Y, n, U und V die in Formel I genannte Bedeutung haben, mit salpetriger Säure diazotiert, mit Piperidin-2-carbonsäure umgesetzt und mit Acetanhydrid cyclisiert.

g) Zur Herstellung der Verbindungen der Formel I, in der W einen 1,3,4,5,6,7-Hexahydro-2H-isoindol-1,3-dion-2-yl-Rest darstellt, wird eine Verbindung der Formel XV

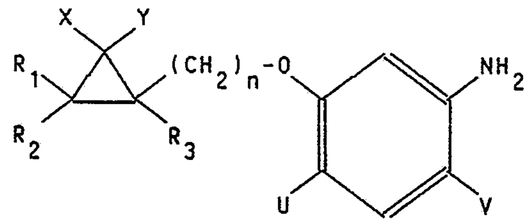

(XV) ,

worin $R_1$, $R_2$, $R_3$, X, Y, n, U und V die in Formel I genannte Bedeutung haben mit einer Verbindung der Formel XVI

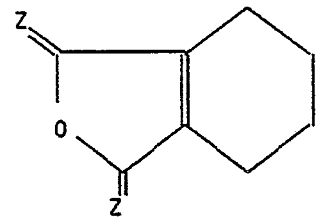

(XVI) ,

in der Z = O oder S bedeutet, umgesetzt.

h) Zur Herstellung der Verbindungen der Formel I, in der W einen 2,3,5,6,7,8-Hexahydro-1H-imidazo-[1,5-a]pyridin-1,3-dion-2-yl-oder einen 2,3,5,6,7,8-Hexahydro-1H-1,3,4-triazolo-[1,2-a]pyridazin-1,3-dion-2-yl-Rest darstellt, wird eine Verbindung der Formel XV

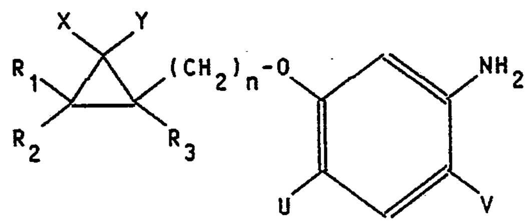

(XV) ,

worin $R_1$, $R_2$, $R_3$, X, Y, n, U und V die in Formel I genannte Bedeutung haben, mit einer Verbindung der Formel XVII oder XVIII

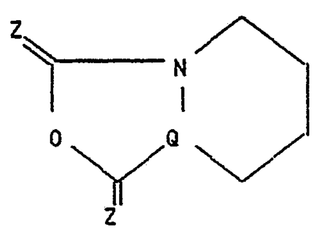

(XVII)

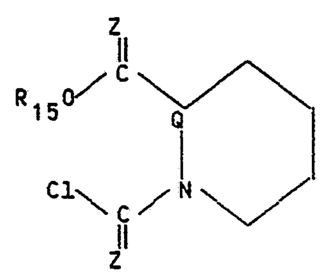

(XVIII)

in denen Z = O oder S, Q = CH oder N und $R_{15}$ und $R_{15}$ $C_{1-4}$-Alkyl bedeuten, umgesetzt oder

eine Verbindung der Formel XV

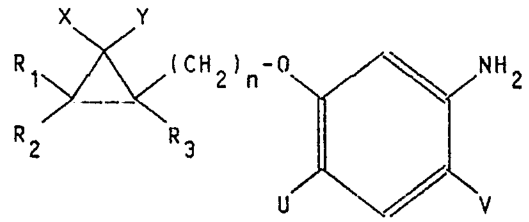

(XV) ,

worin $R_1$, $R_2$, $R_3$, X, Y, n, U und V die in Formel I genannte Bedeutung haben, wird mit Phosgen oder Thiophosgen intermediar zu einer Verbindung der Formel XIX

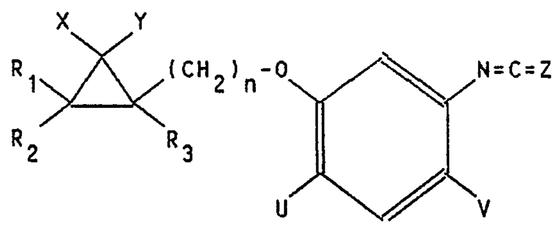

(XIX) ,

worin $R_1$, $R_2$, $R_3$, X, Y, n, U, V und Z die in Formel I genannte Bedeutung haben, umgesetzt und anschließend mit einer Verbindung der Formel XX

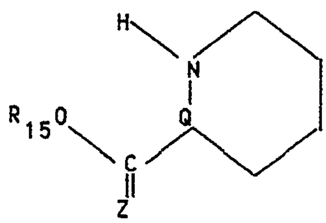

(XX) ,

worin Q = CH oder N, Z = O oder S und $R_{15}$ einen $C_{1-4}$-Alkylrest bedeuten, zur Reaktion gebracht.

i) Eine Verbindung der Formel XXI

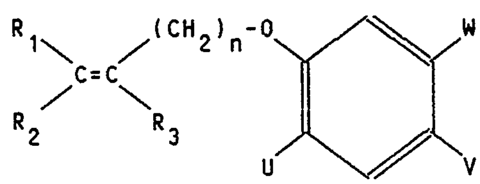

(XXI) ,

in der $R_1$, $R_2$, $R_3$, U, V, W und n die in der Formel I genannte Bedeutung haben, wird mit halogenierten Methanderivaten der Formel XXII

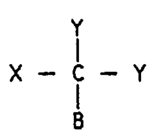

(XXII) ,

in der X und Y die in der Formel I genannte Bedeutung haben und B Wasserstoff oder

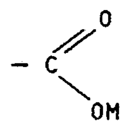

bedeutet, wobei M Alkalimetall darstellt, umgesetzt.

Die Umsetzung gemäß Verfahrensvariante a) wird zweckmäßig unter Zuhilfenahme eines Säureacceptors bei einer Temparatur zwischen 0°C und 150°C, insbesondere zwischen Raumtemperatur und Rückflußtemperatur des Reaktionsgemisches durchgeführt. Als Säureacceptor sind die üblichen basischen Mittel, insbesondere aliphatische Amine, wie z.B. Triethylamin oder Diisopropylamin, aber auch Alkalicarbonate und deren wässrige Lösungen geeignet.

Die Veretherung kann weiterhin in einem Zweiphasensystem unter Verwendung eines Katalysators und gegebenenfalls in Gegenwart von Lösungsmitteln durchgeführt werden. Als Basen dienen Alkalihydroxide oder Alkalicarbonate, fest oder in wässriger Lösung. Als Lösungsmittel geeignet sind die Reaktanden selbst, sofern sie flüssig sind. Ansonsten werden mit Wasser nicht mischbare und gegenüber den Basen inerte Stoffe, wie aliphatische oder aromatische Kohlenwasserstoffe, wie zum Beispiel Hexan, Benzol oder Toluol verwendet. Als Katalysatoren kommen vorzugsweise Kronenether, wie z.B. 1,4,7,10,13,16-Hexaoxacyclooctadecan, und quarternäre Ammoniumsalze in Frage, wie sie in Dehmlow und Dehmlow, Phase Transfer Catalysis, Weinheim 1980, beschrieben sind.

Üblicherweise setzt man die Ausgangsstoffe in stöchiometrischem Verhältnis ein. Ein Überschuß des einen oder anderen kann in Einzelfällen aber durchaus vorteilhaft sein.

Die Umsetzung gemäß den Verfahrensvarianten b) und c) wird zweckmäßig unter Katalyse in einem geeigneten Lösungsmittel durchgeführt. Die Reaktionstemperaturen liegen zwischen Raumtemperatur und 150°C, bevorzugt bei Rückflußtemperatur des Reaktionsgemisches. Als geeignete Lösungsmittel seien beispielsweise genannt Dimethylsulfoxid, Halogenkohlenwasserstoffe, wie zum Beispiel Methylenchlorid und Chloroform, aromatische Kohlenwasserstoffe, wie zum Beispiel Benzol, Toluol, Xylol, Chlorbenzol und Dichlorbenzol, sowie andere gegenüber den Reaktionspartnern inerte Lösungsmittel, wie zum Beispiel Diethylether, Tetrahydrofuran oder Dimethylformamid.

Als Katalysatoren können Säuren wie Essigsäure oder Schwefelsäure, aber auch saure Ionentauscher verwendet werden.

Die Reaktion gemäß Verfahrensvariante d) erfolgt ohne oder unter Verwendung eines geeigneten inerten Lösungsmittels. Als geeignete Lösungsmittel seien beispielsweise genannt Dimethylsulfoxid, Halogenkohlenwasserstoffe, wie z.B. Methylenchlorid oder Chloroform, aromatische Kohlenwasserstoffe, wie z.B. Benzol, Toluol, Xylol, Chlorbenzol und Dichlorbenzol, sowie andere gegenüber den Reaktionspartnern inerte Lösungsmittel, wie z.B. Diethylether, Tetrahydrofuran oder Dimethylformamid.

Die Umsetzung von Verbindungen der Formel IX und Verbindungen der Formel X gemäß Verfahrensvariante e) wird zwechmäßig unter Zuhilfenahme eines Säureacceptors bei einer Temperatur zwischen 0°C und 150°C, insbesondere zwischen Raumtemperatur und Rückflußtemperatur der Lösung durchgeführt. Als Säureacceptor sind die üblichen basischen Mittel, insbesondere aliphatische Amine, wie z.B. Triethylamin oder Diisopropylamin, aber auch Alkalicarbonate und deren wässrige Lösungen geeignet.

Die Alkylierung kann auch in einem Zweiphasensystem unter Verwendung eines Katalysators und gegenbenenfalls in Gegenwart von Lösungsmitteln durchgeführt werden. Als Basen dienen Alkalihydroxide oder Alkalicarbonate, fest oder in wässriger Lösung. Als Lösungsmittel geeignet sind die Reaktanden selbst, sofern sie flüssig sind. Ansonsten werden mit Wasser nicht mischbare und gegenüber den Basen inerte Stoffe, wie aliphatische oder aromatische Kohlenwasserstoffe, wie zum Beispiel Hexan, Benzol oder Toluol verwendet. Als Katalysatoren kommen vorzugsweise Kronenether und quarternäre Ammoniumsalze in Frage.

Überlicherweise setzt man die Ausgangsstoffe in stöchiometrischem Verhältnis ein. Ein Überschuß des einen oder anderen kann in Einzelfällen aber auch durchaus vorteilhaft sein.

Die Umsetzung gemäß Verfahrensvariante f) erfolgt im allgemeinen in drei Stufen ohne zwischenzeitliche Aufreinigung der Produkte. Als Lösungsmittel kann neben Wasser auch jedes inerte organische Lösungsmittel verwendet werden. Es kann aber auch ohne Lösungsmittel gearbeitet werden. Die Reaktionstemperaturen liegen zwischen -20°C und 100°C, bevorzugt zwischen -10°C und Raumtemperatur.

Die Umsetzung gemäß den Verfahrensvarianten g) und h) wird zweckmäßig bei einer Temperatur oberhalb 20°C, z.B. bei 100°C oder bei Rückflußtemperatur der Reaktionsmischung durchgeführt. Falls der Reaktionspartner ein Anhydrid ist wie bei Verbindun gen der Formeln XVI oder XVII, wird die Reaktion zweckmäßig in Gegenwart einer Säure wie Essigsäure durchgeführt, z.B. indem man in Essigsäure als

Lösungsmittel arbeitet. Es ist aber auch möglich, die beiden Reaktionspartner ohne oder unter Verwendung eines inerten Lösungsmittels, wie z.B. Dichlormethan oder Dimethylsulfoxid, zur Reaktion zu bringen und das intermediär entstandene Additionsprodukt mit Säureanhydriden, wie zum Beispiel Acetanhydrid, zu cyclisieren.

Die Umsetzung gemäß Verfahrensvariante h) mit den Verbindungen der Formeln XIX und XX erfolgt in einem inerten Lösungsmittel bei Temperaturen zwischen 20°C und 150°C, bevorzugt bei der Siedetemperatur des Lösungsmittels. Geeignete Lösungsmittel sind z.B. aliphatische und aromatische, gegebenenfalls chlorierte Kohlenwasserstoffe, wie Petrolether, Benzol, Toluol, Xylol, Benzin, Dichlormethan, Chloroform, Tetrachlormethan, 1,2-Dichlorethan, Chlorbenzol, sowie Ether, wie Diethyl-und Di-n-butylether, Methyl-tert.-butylether, Tetrahydrofuran, Diozan, Ketone, wie beispielsweise Aceton, Methlethylketon, Methylisopropylketon, ferner Nitrile, wie Acetonitril und Propiontril, und Sulfoxide, wie Dimethylsulfoxid, oder Sulfolan.

Die Umsetzung gemäß Verfahren i) wird zweckmäßigerweise in einem gegenüber den Reaktionspartnern inerten Lösungsmittel, wie Chloroform, Ethern, wie Dioxan oder Diethylenglykoldialkylethern, aliphatischen Kohlenwasserstoffen, wie Cyclohexan oder Dekan, gegebenenfalls unter Einwirkung einer starken Base, wie Alkalialkoholaten oder -hydroxiden, bevorzugt im 2-Phasensystem unter Zuhilfenahme eines Phasentransfer-Katalysators, wie sie in DEHMLOW UND DEHMLOW: Phase Transfer Catalysis, Weinheim 1980, beschrieben sind, bei Temperaturen zwischen Raumteperatur und 200°C, bevorzugt bei der Siedetemperatur des Lösungsmittels durchgeführt.

Die nach oben genannten Verfahren hergestellten erfindungsgemäßen Verbindungen können nach den üblichen Verfahren aus dem Reaktionsgemisch isoliert werden, beispielsweise durch Abdestillieren des eingesetzten Lösungsmittels bei normalem oder vermindertem Druck oder durch Extraktion.

Ein erhöhter Reinheitsgrad kann in der Regel durch säulenchromatographische Aufreinigung sowie durch fraktionierte Destillation erhalten werden.

Die erfindungsgemäßen Verbindungen stellen in der Regel kristalline oder zähflüssige Substanzen dar, die zum Teil gut löslich in halogenierten Kohlenwasserstoffen, wie Chloroform, Sulfoxiden, wie Dimethylsulfoxid, oder Estern, wie Essigester, sind.

Die erfindungsgemäßen Verbindungen können ein oder mehrere asymmetrische C-Atome enthalten. Die vorliegende Erfindung umfaßt die optisch aktiven Formel und Mischungen davon. In den Beispielen wurden, sofern nicht anders angegeben, die Racemate erhalten.

Die Ausgangsmaterialien der Formeln IV und XV können nach an sich bekannten Verfahren gewonnen werden. Einen möglichen Syntheseweg zeigt das folgende Formelschema:

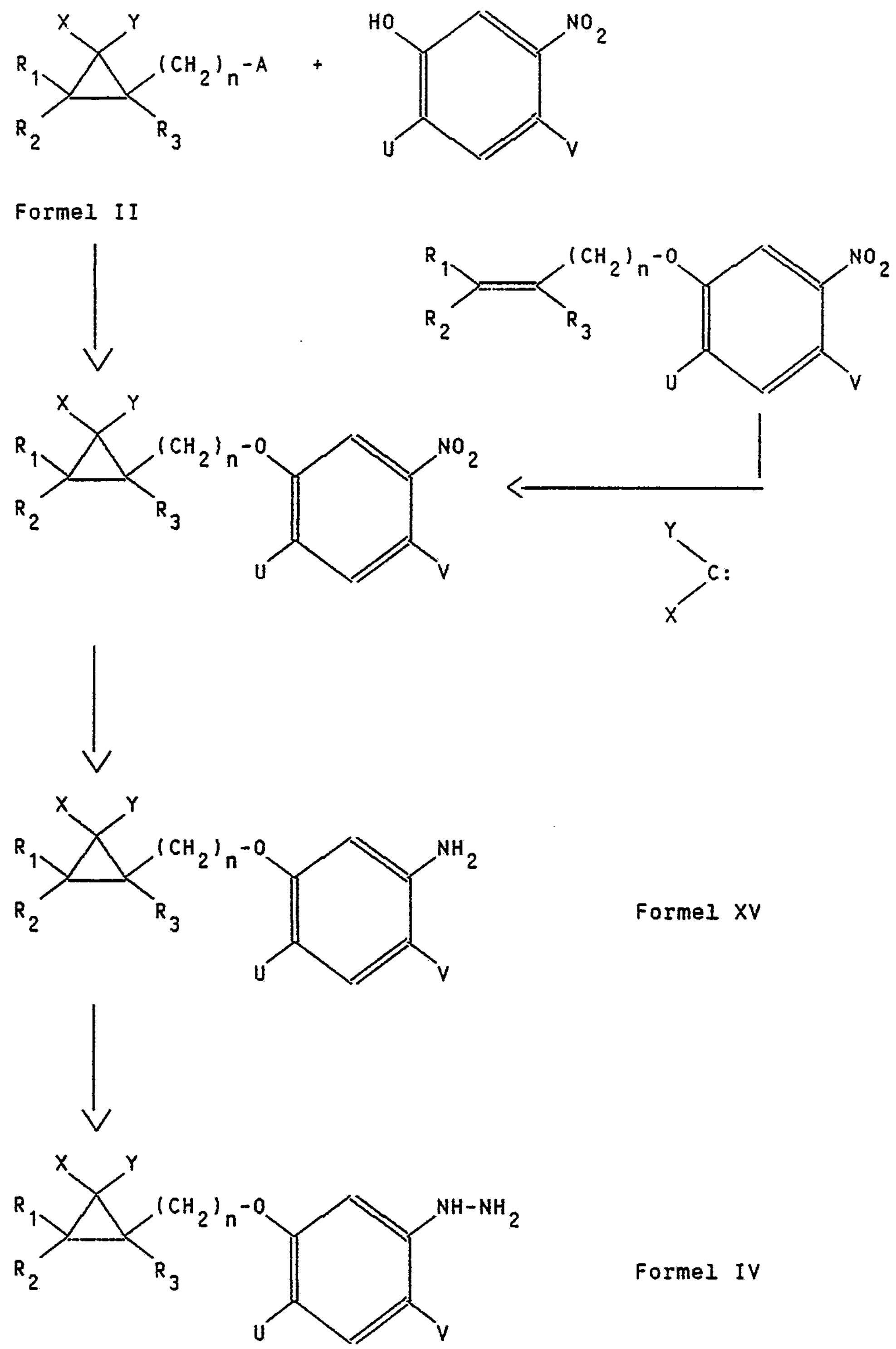

Formel II

Formel XV

Formel IV

Sofern die Herstellung der übrigen genannten Ausgangsmaterialien nicht beschrieben ist, sind diese bekannt oder analog zu bekannten Verfahren herstellbar.

Die erfindungsgemäßen Verbindungen weisen eine ausgezeichnete herbizide Wirksamkeit gegen wirtschaftlich wichtige mono-und dikotyle Unkräuter auf. Dabei ist es gleichgültig, ob die Substanzen in Vorsaat-, Vorauflauf-oder Nachauflaufspritzung ausgebracht werden.

Überraschenderweise zeigen die erfindungsgemäßen Verbindungen sehr gute Verträglichkeiten für Reis, Weizen, Soja, Mais, Baumwolle und Gerste.

Die erfindungsgemäßen Verbindungen können z.B. zur Unkrautbekämpfung gegen die folgenden Pflanzengattungen eingesetzt werden:

Dikotyle Unkräuter der Gattungen Abutilon, Chrysanthemum, Brassica, Helianthus, Mentha, Sinapis,

Lepidium, Galium, Stellaria, Anthemis, Chenopodium, Atriplex, Senecio, Portulaca, Ipomea, Matricaria, Galinsoga, Urtica, Amaranthus, Polygonum, Sesbania, Ambrosia, Sonchus, Solanum, Lamium, Veronica, Datura, Viola, Centaurea und Galeopsis.

Monokotyle Unkräuter der Gattungen Avena, Alopecurus, Echinochloa, Setaria, Panicum, Digitaria, Poa, Eleusine, Brachiaria, Lolium, Bromus, Cyperus, Agropyron, Saggitaria, Cynodon, Monochoria, Fimbristylis, Eleocharis, Ischaemum und Apera.

Die Verwendung der erfindungsgemäßen Verbindungen ist keineswegs auf die genannten Unkrautgattungen und Kulturpflanzen beschränkt, sondern erstreckt sich in gleicher Weise auch auf andere Pflanzen.

Die Verbindungen eignen sich in höheren Aufwandmengen zur Totalunkrautbekämpfung, wie z.B. in Industrie-und auf Gleisanlagen und auf Wegen und Plätzen. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht-und Hopfenanlagen, eingesetzt werden.

Die angewandte Wirkstoffmenge kann in einem größeren Bereich variiert werden. Sie hängt im wesentlichen von der Art des gewünschten Effekts ab. Im allgemeinen liegen die Aufwandmengen zwischen 0,01 und 5 kg Wirkstoff pro Hektar Bodenfläche, vorzugsweise z.B. bei der Unkrautbekämpfung zwischen, 0,1 und 0,5 kg pro ha.

Sofern eine Verbreiterung des Wirkungsspektrums oder Wirkungssteigerung beabsichtigt ist, können auch andere Herbizide zugesetzt werden. Beispielsweise eigenen sich als herbizid wirksame Mischungspartner Wirkstoffe, die in Weed Abstracts, Vol: 35, No. 3, 1986, unter dem Titel "List of common names and abbreviations employed for currently used herbicides and plant growth regulators in Weed Abstracts" aufgeführt sind.

Zweckmäßig werden die erfindungsgemäßen Verbindungen oder deren Mischungen in Form von Zubereitungen, wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier-und/oder Dispergierhilfsmitteln angewandt.

Geeignete flüssige Trägerstoffe sind zum Beispiel aliphatische und aromatische Kohlenwasserstoffe wie Toluol, Xylol, Cyclohexanon, Isophoron, Dimethylformamid, weiterhin Mineralölfraktionen und Pflanzenöle.

Als feste Trägerstoffe eignen sich Mineralien, zum Beispiel Gips, Tonsil, Silicagel, Talkum, Kaolin, Attapulgit, Kalkstein und pflanzliche Produkte, wie zum Beispiel Mehle.

An oberflächenaktiven Stoffen sind zum Beispiel zu nennen Calciumligninsulfonat, Polyethylenalkylphenylether, Polyoxyethy len-sorbitester, Polyoxyethylen-polypropylen-polymere, Naphthalinsulfonsäuren und deren Salze, kationische Tenside mit tert. Aminen, die teilweise durch Polyethoxy-Gruppen substituiert sind, Phenolsulfonsäuren und deren Salze, Formaldehydkondensate, Fettalkoholsulfate sowie substituierte Benzolsulfonsäure und deren Salze.

Der Anteil des bzw. der Wirkstoffe(s) in den verschiedenen Zubereitungen kann in weiten Grenzen variieren. Beispielsweise enthalten die Mittel etwa 10 bis 90 Gewichtsprozent Wirkstoff, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, wie zum Beispiel mit Wasser als Träger in Spritzbrühmengen von etwa 100 bis 3000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, wie durch Mahl-oder Mischverfahren, erfolgen. Gewünschtenfalls können die Einzelkomponenten auch erst kurz vor ihrer Verwendung gemischt werden, wie es zum Beispiel im sogenannten Tankmixverfahren in der Praxis gemacht wird.

Zur Herstellung der Zubereitungen werden zum Beispiel die folgenden Bestandteile eingesetzt:

a1) **Spritzpulver**

80 Gewichtsprozent Wirkstoff
10 Gewichtsprozent Kaolin
2 Gewichtsprozent Natriumsalz des N-Methyl-N-oleyltaurins
8 Gewichtsprozent Calciumsalz der Ligninsulfonsäure

a2) **Spritzpulver**

20 Gewichtsprozent Wirkstoff
35 Gewichtsprozent Bentonit
8 Gewichtsprozent Calciumsalz der Ligninsulfonsäure
2 Gewichtsprozent Natriumsalz des N-Methyl-N-oley-taurins
35 Gewichtsprozent Kieselsäure

b) **Paste**

45 Gewichtsprozent Wirkstoff
5 Gewichtsprozent Natriumaluminiumsilikat
15 Gewichtsprozent Cetylpolyglycolether mit 8 Mol Ethylenoxid
2 Gewichtprozent Spindelöl
10 Gewichtsprozent Polyethylenglycol
23 Gewichtsprozent Wasser

c) **Emulsionskonzentrat**

20 Gewichtsprozent Wirkstoff
75 Gewichtsprozent Isophorn
2 Gewichtsprozent Calciumdodecylphenylsulfonat
3 Gewichtsprozent Fettalkoholpolyglycolether

Die folgenden Beispiele erläutern die Herstellung der erfindungsgemäßen Verbindungen:

Herstellungsbeispiel 1

N-[4-Chlor-5-(2,2-dichlorcyclopropylmethoxy)-2-fluorphenyl]-3,4,5,6-tetrahydrophthalsäureimid (Verbindung Nr. 1.1)

8,9 g (0,031 Mol) 4-Chlor-2-fluor-5-(2,2-dichlorcyclopropylmethoxy)-anilin werden in 12,5 ml Eisessig gelöst und 3,85 g 3,4,5,6-Tetrahydrophthalsäureanhydrid zugegeben. Es wird 3 Stunden unter Rückfluß erhitzt, anschließend auf Eiswasser gegossen und mit Ether extrahiert. Die Etherphase wird mit Natriumhydrogencarbonatlösung neutralisiert, mit gesättigter Kochsalzlösung gewaschen, über Magnesiumsulfat getrocknet und das Lösungsmittel abgezogen. Der Rückstand wird über Kieselgel chromatografiert, wobei mit einem Gemisch von 9 Teilen Hexan und 1 Teil Essigester eluiert wird.

Es wurden 7,8 g = 75 % der Theorie bräunliche Kristalle mit einem Schmelzpunkt von 121-122°C erhalten.

Analog wurden folgende Verbindungen erhalten:

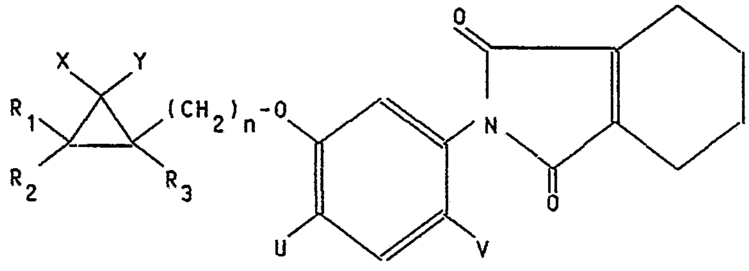

| Verbindung Nr. | $R_1$ | $R_2$ | $R_3$ | n | X | Y | U | V | Physikalische Konstante |
|---|---|---|---|---|---|---|---|---|---|
| 1.2 | H | H | H | 1 | F | F | Cl | F | $n_D^{25}$ = 1,5375 |
| 1.3 | H | H | H | 2 | F | F | Cl | F | $n_D^{24}$ = 1,5280 |
| 1.4 | H | H | H | 2 | Cl | Cl | Cl | F | $n_D^{24}$ = 1,5555 |

Herstellungsbeispiel 2

2-[4-Chlor-5-(2,2-difluorcyclopropylmethoxy)-2-fluorphenyl]-4,5,6,7-tetrahydro-(2H)-1,2,3-triazolo[3,4-a]-pyridin-8-ium-3-olat (Verbindung Nr. 2.1)

4,6 g 4-Chlor-5-(2,2-difluorcyclopropylmethoxy)-2-fluoranilin werden in einer Mischung aus 9,7 ml konzentrierter Salzsäure und 41,5 ml Wasser suspendiert und auf -10°C gekühlt. Dann wird tropfenweise eine Lösung von 1,38 g Natriumnitrit in 4,2 ml Wasser so zugegeben, daß die Temperatur der Reaktionsmischung -5°C nicht übersteigt. Nach beendeter Zugabe wird 1 Stunde bei -5°C nachgerührt und überschüssige salpetrige Säure mit Harnstoff zerstört, bis der Test mit Jodstärkepapier negativ ist. Die so erhaltene Lösung wird auf 0°C erwärmt und zu einer eiskalten Lösung von 2,3 g Pipecolinsäure und 8,3 ml Triethylamin in 27,7 ml Wasser getropft. Nach beendeter Zugabe wird 1 Stunde bei 0°C nachgerührt und die Reaktionsmischung mit Methylenchlorid mehrfach extrahiert. Die organischen Phasen werden vereinigt, über Magnesiumsulfat getrocknet und das Lösungsmittel abgedampft. Der Rückstand wird in 40 ml Ether aufgenommen und miot 4,14 ml Essigsäureanhydrid und 2,07 ml Pyridin versetzt. Über Nacht wird bei Raumtemperatur gerührt, anschließend auf Eiswasser gegeossen und mit Essigester extrahiert. Die organischen Phasen werden über Magnesiumsulfat getrocknet und das Lösungsmittel abgedampft. Der Rückstand über Kieselgel mit einem Ela tionsmittel (95 Teile Essigester/5 Teile Methanol) chromatografiert.

Es werden 0,9 g = 13,4 % der Theorie Kristalle erhalten vom Schmelzpunkt 125-127°C.

Herstellungsbeispiel 3

3-Chlor-2-[4-Chlor-5-(2,2-difluorcyclopropylmethoxy)-2-fluorphenyl]-4,5,6,7-tetrahydro-2H-indazol (Verbindung Nr. 3.1)

5 g 4-Chlor-5-(2,2-difluorcyclopropylethoxy)-2-fluorphenylhydrazin werden in 18 ml Eisessig gelöst, 2,85 ml Cyclohexanon-2-carbonsäureethylester zugegeben und die Reaktionsmischung 8 Stunden lang unter Rückfluß erhitzt. Anschließend wird die Mischung auf Wasser gegeben und mit Methylenchlorid extrahiert. Die organischen Phasen werden mit Kaliumhydrogencarbonatlösung neutralisiert, mit gesättigter Kochsalzlösung gewaschen und über Magnesiumsulfat getrocknet. Nach dem Abdampfen der Lösungsmittels erhält man 8,4 g = 100 % d.Th. an Rohprodukt. Das Rohprodukt wird mit 3,6 ml Phosphoroxychlorid versetzt und die Mischung unter Rückfluß erhitzt. Nach 4 Stunden wird abgekühlt, mit Methylenchlorid aufgenommen, mit Wasser und anschließend mit gesättigter Sodalösung gewaschen, über Magnesiumsulfat getrocknet und das Lösungsmittel abgedampft. Der Rückstand wird über Kieselgel mit einer Mischung aus 95 Teilen Dichlormethan und 5 Teilen Methanol chromatografiert.

Man erhält 2,6 g = 35,6 % der Theorie braunes Öl mit einem Brechungsindex $n_D^{25}$ = 1,5345.

Analog wurden die folgenden Verbindungen erhalten:

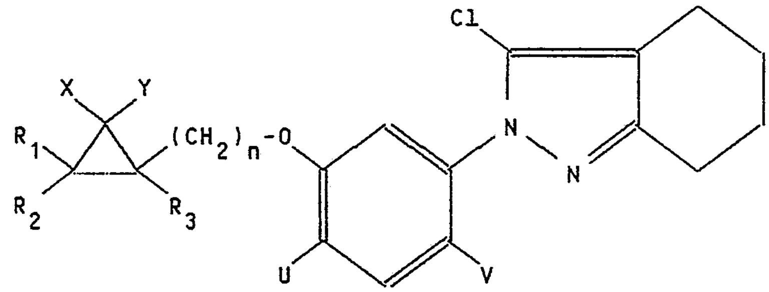

| Verbindung Nr. | $R_1$ | $R_2$ | $R_3$ | n | X | Y | U | V | Physikalische Konstante |
|---|---|---|---|---|---|---|---|---|---|
| 3.2 | H | H | H | 1 | F | F | Cl | F | $n_D^{20}$ = 1,538 |
| 3.3 | H | H | H | 1 | Cl | Cl | Cl | F | $n_D^{25}$ = 1,5713 |
| 3.4 | H | H | H | 2 | Cl | Cl | Cl | F | $n_D^{25}$ = 1,5615 |

Herstellungsbeispiel 4

2-[4-Chlor-5-(2,2-difluorcyclopropylmethoxy)-2-fluorphenyl]-2,3,5,6,7,8-hexahydro-1,2,4-triazolo-[4,3-a]-pyridin-3-on (Verbindung Nr. 4.1)

3 g 4-Chlor-5-(2,2-difluorcyclopropylmethoxy)-2-fluorphenylhydrazin werden in 40 ml Xylol gelöst, 2,4 g 1-Ethoxycarbonyl-2-piperidon und 1 g Phosphorpentoxid zugefügt und die Mischung 3 Stunden unter Rückfluß erhitzt. Anschließend wird auf 100 ml Wasser gegeben und die organische Phase abgetrennt. Die organische Phase wird mit Kaliumhydrogencarbonatlösung entsäuert, über Magnesiumsulfat getrocknet und das Lösungsmittel abgezogen. Der Rückstand wird über Kieselgel mit Essigester als Eluationsmittel chromatografiert.

Man erhält 1,2 g = 23% der Theorie eines Öls mit dem Brechungsindex $n_D^{20}$ = 1,5438

Analog wurden die folgenden Verbindungen erhalten:

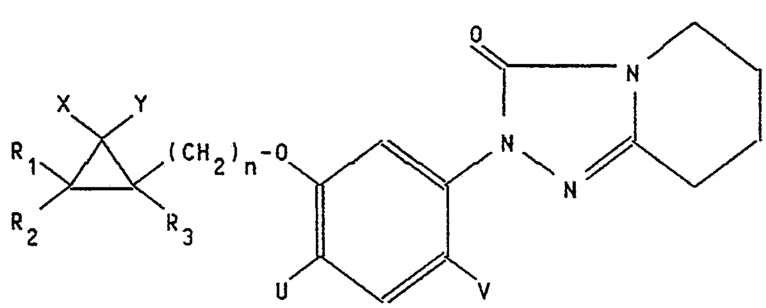

| Verbindung Nr. | $R_1$ | $R_2$ | $R_3$ | n | X | Y | U | V | Physikalische Konstante |
|---|---|---|---|---|---|---|---|---|---|
| 4.2 | H | H | H | 1 | Cl | Cl | Cl | F | $n_D^{25}$ = 1,5655 |
| 4.3 | H | H | H | 2 | Cl | Cl | Cl | F | $n_D^{25}$ = 1,5620 |
| 4.4 | H | H | H | 2 | F | F | Cl | F | $n_D^{25}$ = 1,5332 |

Herstellungsbeispiel 5

2-[4-Chlor-5-(2,2-difluorcyclopropylmethoxy)-2-fluorphenyl-3 thioxo-2,3,5,6,7,8-hexahydro-1H-imidazo[1,5-a]-pyridin-1-on (Verbindung Nr. 5.1)

2,9 g 4-Chlor-5-(2,2-dicfluorcyclopropylmethoxy)-2-fluorphenyl-isothiocyanat werden in 20 ml Hexan gelöst und zu einer Lösung von 1,5 ml Piperidin-2-carbonsäureethylester in 10 ml Hexan zugetropft. Die Mischung wird 2 Stunden unter Rückfluß erhitzt. Anschließend wird das Lösungsmittel abgezogen und der Rückstand über Kieselgel mit einer Mischung aus 3 Teilen Hexan und 1 Teil Essigester chromatografiert.

Ma erhält 3,2 g = 82 % der Theorie eines zähflüssigen Öls, dessen Brechungsindex $n_D^{40}$ bei 1,54 liegt.

Herstellungsbeispiel 6

2-[4-Chlor-5-(2,2-dichlorcyclopropylmethoxy)-2-fluorphenyl]-2,3,5,6,7,8-hexahydro-1H-imidazo-[1,5-a]pyridin-1,3-dion (Verbindung Nr. 6.1)

Analog Herstellungsbeispiel 5 erhält man bei Einsatz von 8,33 g 4-Chlor-2-fluor-5-(2,2-dichlorcyclopropylmethyl)-phenylisocyanat und 4,69 ml Piperidin-2-carbonsäureethylester in 100 ml Hexan 7,7 g = 6 % der Theorie eines halbkristallinen Öls, dessen Kernresonanzspektrum folgende Daten liefert:
$\delta$ = 1,1 - 2,4 ppm (m) 9 H, $\delta$ = 2,7 - 3,15 ppm (m) 1H,
$\delta$ = 3,85 - 4,4 ppm (m) 4 H, $\delta$ -6,87 (d 6 Hz) 1H,
$\delta$ = 7,3 (d 9Hz) 1H

Herstellungsbeispiel 7

3-[4-Chlor-5-(2,2-difluorcyclopropylmethoxy)-2-fluorphenyl]-5-tert.-butyl-1,3,4-oxdiazolin-2-(3H)-on (Verbindung Nr. 7.1)

3 g 4-Chlor-5-(2,2-difluorcyclopropylmethoxy)-2-fluorphenylhydrazin-hydrochlorid werden in 18,5 ml Toluol suspendiert, mit 3,6 ml Triäthylamin versetzt und die Mischung bis zum Auflösen des Hydrochlorids gerührt. Dann wird ohne Kühlung langsam 1,6 ml Pivalinsäurechlorid zugetropft und die Mischung 2 Stunden bei Raumtemperatur nach gerührt. Die Reaktionsmischung wird anschließend mit Wasser, gesättigter Kaliumbicarbonatlösung und noch einmal mit Wasser gewaschen, über Magnesiumsulfat getrocknet und das Lösungsmittel abgezogen. Als Rückstand verbleiben 3,7 g eines rotes Öls, das neben Verunreinigungen N'-[4-Chlor-5-(2,2-difluorcyclopropylmethoxy)-2-fluorphenyl]-pivalinsäurehydrazid enthält. Dieses Rohprodukt wird in 22 ml einer 20%igen Phosgenlösung in Toluol gelöst, langsam auf 100°C erhitzt und 3 Stunden bei dieser Temperatur belassen. Anschließend wird abge kühlt, mit 20 ml Methanol versetzt

und eingeengt. Der Rückstand wird in Methylenchlorid aufgenommen, zweimal mit Kaliumbicarbonatlösung und einmal mit Wasser gewaschen, über Magnesiumsulfat getrocknet und eingeengt. Zur Aufreinigung wird das Rohprodukt über Kieselgel mit dem Laufmittel Hexan/Essigester - 4 : 1 chromatografiert.

Man erhält 1,2 g = 24,5 % der Theorie Produkt mit einem Brechungsindex $n_D^{20}$ = 1,5082.

Herstellungsbeispiel 8

N-[4-Chlor-5-(2,2-difluorcyclopropylmethoxy)-2-fluorphenyl]-5,6,7,8-tetrahydrophthalsäureimid (Verbindung 8.1)

1,2 g N-(4-Chlor-2-fluor-5-vinyloxphenyl)-5,6,7,8-tetrahydrophthalsäureimid wird in 5 ml Diäthylenglykoldimethyläther gelöst, die Lösung zum Rückfluß erhitzt und dann langsam die Lösung von 2,8 g Natrium-chlordifluoracetat in 20 ml Diäthylenglykoldimethyläther zugegeben. Anschließend wird noch 5 Stunden zum Rückfluß erhitzt, dann vom gebildeten Kochsalz abgetrennt und das Lösungsmittel abgezogen. Der Rückstand wird chromatografisch über Kieselgel mit einer Mischung von Hexan/Diäthyläther = 1:1 gereinigt.

Man erhält 0,6 g = 43 % der Theorie einer kristallinen Substanz mit einem Schmelzpunkt von 105-107°C.

Herstellungsbeispiel 9

1-[4-Chlor-5-(2,2-difluorcyclopropylmethoxy)-2-fluorphenyl]-4-difluormethyl-3-methyl-1,2,4-triazolin-5-(4H)-on (Verbindung Nr. 9.1)

8,2 g 4-Chlor-5-(2,2-difluorcyclopropylmethoxy)-2-fluorphenyl]-hydrazin-hydrochlorid werden in 163 ml Wasser gelöst, mit 81 ml Äthanol und Tropfenweise mit einer Lösung von 2,75 ml Brenztraubensäure in 81 ml Wasser vesetzt. Eine Stunde wird nachgerührt und das ausgefallene Produkt abfiltriert und getrocknet. Man erhält 7,9 g des noch leicht verunreinigten Hydrazons, das in dieser Form eingesetzt wird.

Das Hydrazon wird in 130 ml Toluol gelöst, mit 3,3 ml Triäthylamin versetzt und bis zum Aufklaren der Lösung erwärmt. Dann wird auf ca. 35°C abgekühlt, 5,1 ml Phosphorsäurediphenylesteraxid tropfenweise zugegeben und langsam auf 75°C erwärmt. Diese Temperatur wird solange beibehalten, bis kein Stickstoff mehr entweicht, anschließend wird zum Rückfluß erhitzt. Nach 12 Stunden wird abgekühlt, die Reaktionslösung dreimal mit 10%iger Natronlauge extrahiert, die wäßrige Phase zweimal mit Toluol gewaschen und angesäuert. Das Produkt wird mit Toluol extrahiert und die organische Phase getrocknet. Nach dem Abziehen des Lösungsmittels wird das Produkt über Kieselgel mit Essigester chromatografiert. Man erhält 3,7 g = 35 % der Theorie 1-[4-Chlor-5-(2,2-difluorcyclopropylmethoxy)-2-fluorphenyl]-3-methyl-1,2,4-triazolin-5-(4H)-on.

Das nach obiger Vorschrift erhaltene Pordukt wird in 300 ml Cyclohexan gelöst, mit 2,7 g Kaliumhydroxid und 2 g Tetrabutylammoniumbromid versetzt, zum Rückfluß erhitzt und dann Chlordifluormethan eingeleitet. Nach Beendigung der Reaktion (Kontrolle mittels Dünnschichtchromatografie) wird die Lösung dekantiert, je zweimal mit 1n Salzsäure und 10%iger Natronlauge gewaschen, über Magnesiumsulfat getrocknet und eingeengt. Der Rückstand wird über Kieselgel mit Essigester und anschließend noch einmal mit Methylenchlorid chromatografiert.

Man erhält 80 mg = 2 % der Theorie des Produktes mit einem Brechungsindex $n_D^{40}$ =m 1,535.

Herstellung von einigen Ausgangsmaterialien

Darstellung von 4-Chlor-5-(2,2-difluorcyclopropylmethoxy)-2-fluornitrobenzol

3,6 g 2-Chlor-4-fluor-5-nitrophenol werden in Dimethylformamid gelöst und mit Kaliumcarbonat versetzt. Eine Stunde wird bei Raumtemperatur nachgerührt und dann 3,3 g 2,2-Difluorcyclopropylmethylbromid zugegeben. Anschließend wird über zwei Stunden auf 80°C erhitzt und dann auf Wasser gegeben. Die Mischung wird mit Methylenchlorid extrahiert und die organische Phase mit gesättigter Kochsalzlösung gewaschen, über Magnesiumsulfat getrocknet und eingeengt. Der Rückstand wird über Kieselgel mit einer Mischung aus 3 Teilen Hexan und 1 Teil Essigester chromatografiert. Man erhält 5 g = 93 % der Theorie

gelbe Kristalle, deren Kernresonanzspektrum folgende Daten liefert:
$\delta$ = 1,1 - 2,4 ppm (m) 3 H, $\delta$ = 4,15 ppm (d 7 Hz,tr 1,5 Hz) 2H,
$\delta$ = 7,35 ppm (d 10 Hz) 1 H, $\delta$ = 7,5 ppm (d 7 Hz) 1 H

Analog wurden die folgenden Verbindungen erhalten:

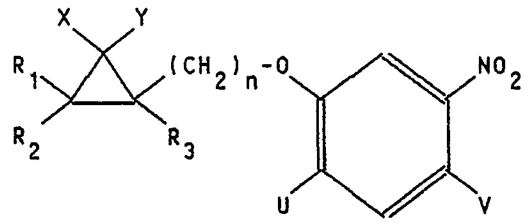

| $R_1$ | $R_2$ | $R_3$ | X | Y | n | U | V |
|---|---|---|---|---|---|---|---|
| H | H | H | F | F | 2 | Cl | F |
| H | H | H | Cl | Cl | 1 | Cl | F |
| H | H | H | Cl | Cl | 2 | Cl | F |

Darstellung von 4-Chlor-2-fluor-5-(2,2-difluorcyclopropylmethoxy)-anilin

36,9 g 4-Chlor-2-fluor-5-(2,2-difluorcyclopropylmethoxy)-nitrobenzol werden in 600 ml Ethanol und unter Zusatz von 5 g 10%-igem Palladiummetall auf Aktivkohle bei Raumtemperatur und Normaldruck hydriert. Nachdem die Wasserstoffaufnahme beendet ist, wird der Katalysator abfiltriert, das Lösungsmittel abgedampft und man erhält 29,3 g = 89 % der Theorie eines Öls, dessen Kernresonanzspektrum folgende Daten liefert:
$\delta$ = 1,1 - 2,2 ppm (m) 3 H, $\delta$ = 4,0 ppm (d 10 Hz breit) 2 H,
$\delta$ = 5 ppm (s breit) 2 H, $\delta$ = 6,5 ppm (d 7 Hz) 1H,
$\delta$ = 7,0 ppm (d 10 Hz) 1 H

Analog wurden die folgenden Verbindungen erhalten:

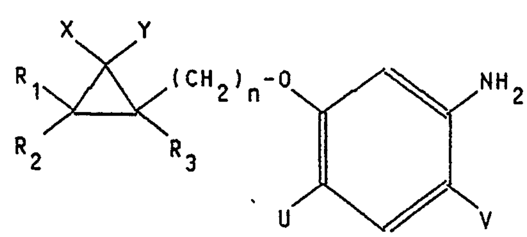

| $R_1$ | $R_2$ | $R_3$ | X | Y | n | U | V |
|---|---|---|---|---|---|---|---|
| H | H | H | F | F | 2 | Cl | F |
| H | H | H | Cl | Cl | 1 | Cl | F |
| H | H | H | Cl | Cl | 2 | Cl | F |

Darstellung von 4-Chlor-2-fluor-5-(2,2-difluorcyclopropylmethoxy)-phenylhydrazin

29,3 g 4-Chlor-2-fluor-5-(2,2-difluorcyclopropylmethoxy)-anilin werden mit 45 ml halbkonzentrierten Salzsäure vorgelegt und mit 273 ml konzentrierter Salzsäure versetzt. Dann wird auf -10°C abgekühlt und tropfenweise mit einer Lösung von 8,3 g Natriumnitrit in 17 ml Wasser versetzt, so daß die Temperatur -5°C nicht übersteigt. 3 Stunden wird bei -5°C nachgerührt und dann bei -15°C eine Lösung von 57,3 g Zinn-(II)-chloriddihydrat in 34 ml konzentrierter Salzsäure so zugetropft, daß -10°C nicht überschritten werden. Über Nacht wird auf Raumtemperatur erwärmt. Zur Aufarbeitung wird auf 0°C abgekühlt und langsam mit 430 ml 32%iger Natronlauge neutralisiert. Der ausgefallene Zinnhydroxidschlamm wird durch Filtrieren mit Celite entfernt, der Filterkuchen mit Ether gewaschen und die wäßrige Mutterlauge mehrfach mit Ether extrahiert. Die vereinigten organischen Phasen werden über Magnesiumsulfat getrocknet und das Lösungsmittel abgezogen.

Man erhält 26 g = 100 % der Theorie Öl, dessen Kernresonanzspektrum folgende Daten liefert:
$\delta$ = 1,1 - 2,3 ppm (m) 3 H, $\delta$ = 4,0 ppm (d 8 Hz breit) 2 H,
$\delta$ = 6,85 ppm (d 7 Hz) 1 H, $\delta$ = 7,0 ppm (d 10 Hz) 1 H

Analog wurden die folgenden Verbindungen erhalten:

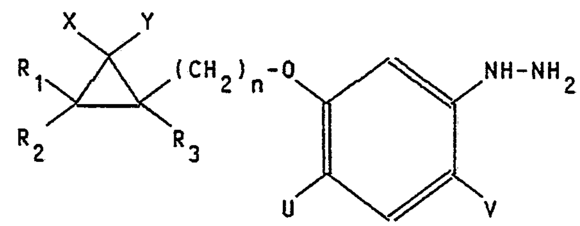

| $R_1$ | $R_2$ | $R_3$ | n | X | Y | U | V |
|---|---|---|---|---|---|---|---|
| H | H | H | 2 | F | F | Cl | F |
| H | H | H | 1 | Cl | Cl | Cl | F |
| H | H | H | 2 | Cl | Cl | Cl | F |

Die nachfolgenden Beispiele erläutern die Wirksamkeit der erfindungsgemäßen Verbindungen.

Anwendungsbeispiel A

Im Gewächshaus wurden die erfindungsgemäßen Verbindungen in einer Aufwandmenge von 1 kg Wirkstoff/ha, emulgiert in 500 l Wasser/ha, auf Testpflanzenarten der Gattungen Matricaria und Viola im Vor- und Nachauflaufverfahren gespritzt. 3 Wochen nach der Behandlung wurde das Behandlungsergebnis bonitiert, wobei alle Verbindungen gemäß den Herstellungsbeispielen 1 bis 9 in einem Boniturschema von 0 = keine Wirkung bis 4 = Vernichtung eine Vernichtung der Tespflanzen bewirkten.

Anwendungsbeispiel B

Im Gewächshaus wurden die aufgeführten Pflanzenspezies vor dem Auflaufen mit den aufgeführten Verbindungen in einer Aufwandmenge von 0,1 kg Wirkstoff/ha behandelt. Die Verbindungen wurden zu diesem Zweck als Emulsion mit 500 Litern Wasser/ha gleichmäßig über die Pflanzen versprüht. 3 Wochen nach der Behandlung zeigten die erfindungsgemäßen Verbindungen im Vergleich zu bekannten Mitteln eine hohe Selektivität bei ausgezeichneter Wirkung gegen das Unkraut.

| Verbindung | Br | So | Go | Gl | St | Ab | Ma | Vi | Av | Al | Ec | Se | Cy |
|---|---|---|---|---|---|---|---|---|---|---|---|---|---|
| Nr. 4.1 | 4 | 4 | 0 | 0 | 4 | 4 | 4 | 4 | 4 | 4 | 4 | 4 | 4 |
| Vergleichsmittel | | | | | | | | | | | | | |
| EP 61 741 | | | | | | | | | | | | | |
| Nr. 1 | 2 | 3 | 1 | 1 | 3 | 3 | 4 | 4 | 2 | 2 | 2 | 4 | 1 |
| EP 105 721 | | | | | | | | | | | | | |
| Nr. 1 | 3 | 4 | 2 | 1 | 3 | 4 | 4 | 4 | 2 | 3 | 4 | 4 | 0 |
| Oxadiazon | 0 | 3 | 0 | 0 | 2 | 3 | 2 | 4 | 0 | 2 | 1 | 3 | 0 |

Boniturschema

0 = keine Wirkung
1 = schwache Schädigung
2 = mittlere Schädigung
3 = starke Schädigung
4 = total vernichtet

Species

Br = Brassica ssp.
So = Solanumn ssp.
Go = Gossypium hirsutum
Gl = Glycine max
St = Stellaria media
Ab = Abutilon hybridum
Ma = Matricaria chamomilla
Vi = Viola tricolor
Av = Avena fatua

Al = Alopecurus myosuroides
Ec = Echinochloa crus-galli
Se = Setaria italica
Cy = Cyperus esculentus

Anwendungsbeispiel C

Im Gewächshaus wurden die aufgeführten Pflanzenspezies nach dem Auflaufen mit den aufgeführten Verbindungen in einer Aufwandmenge von 0,3 kg Wirkstoff/ha behandelt. Die Verbindungen wurden zu diesem Zweck als Emulsion mit 500 Litern Wasser/ha gleichmäßig über die Pflanzen versprüht. Hier zeigten 3 Wochen nach der Behandlung die erfindungsgemäßen Verbindungen im Vergleich zu der bekannten Verbindung eine hohe Selektivität bei ausgezeichneter Wirkung gegen das Unkraut.

| Verbindung | Br | So | Gl | He | St | Ab | Ma | Vi | Ch | Ip | Ze | Tr | Ho | Or | Sr | Se |
|---|---|---|---|---|---|---|---|---|---|---|---|---|---|---|---|---|
| Nr. 1.1 | 4 | 4 | 0 | 4 | 4 | 4 | 4 | 4 | 4 | 4 | 0 | 0 | 0 | 0 | 0 | 3 |
| Nr. 3.1 | 4 | 4 | 0 | 4 | 2 | 2 | 4 | 3 | 4 | 3 | 0 | 0 | 0 | 0 | 0 | 4 |
| Nr. 3.2 | 4 | 4 | 0 | 3 | 4 | 3 | 4 | 4 | 4 | 4 | 0 | 0 | 0 | 0 | 0 | 4 |
| Nr. 3.4 | 2 | 3 | 0 | 3 | 1 | 1 | 2 | 3 | 3 | 2 | 0 | 0 | 0 | 0 | 0 | 1 |
| Nr. 4.3 | 3 | 4 | 0 | 3 | 3 | 4 | 4 | 3 | 4 | 4 | 0 | 0 | 0 | 0 | 0 | 3 |
| Vergleichsmittel | | | | | | | | | | | | | | | | |
| Oxadiazon | 4 | 4 | 4 | 2 | 2 | 3 | 3 | 4 | 3 | 4 | 2 | 2 | 2 | 2 | 2 | 4 |

Boniturschema

0 = keine Wirkung
1 = schwache Schädigung
2 = mittlere Schädigung
3 = starke Schädigung
4 = total vernichtet

Species

Br = Brassica ssp.
So = Solanumn ssp.
Gl = Glycine max
He = Helianthus annuus
St = Stellaria media
Ab = Abutilon hybridum
Ma = Matricaria chamomilla
Vi = Viola tricolor
Ch = Chrysanthemum segetum

Ip = Ipomoea purpurea
Ze = Zea mays
Tr = Triticum aestivum
Ho = Hordeum vulgare
Or = Oryza sativa
Sr = Sorghum sativum
Se = Setaria italica

Anwendungsbeispiel D

Im Gewächshaus wurden die aufgeführten Pflanzenspezies nach dem Auflaufen mit den aufgeführten Verbindungen in einer Aufwandmenge von 0,3 kg Wirkstoff/ha behandelt. Die Verbindungen wurden zu diesem Zweck als Emulsion mit 500 Litern Wasser/ha gleichmäßig über die Pflanzen versprüht. Hier zeigten 3 Wochen nach der Behandlung die erfindungsgemäßen Verbindungen gute herbizide Eigenschaften.

| Verbindung | Ec | Ce | Cd | Fi |
|---|---|---|---|---|
| Nr. 1.1 | 1 | 1 | 2 | 3 |
| Nr. 1.2 | 4 | 2 | 4 | 4 |
| Nr. 3.2 | 4 | 1 | 3 | 4 |
| Nr. 4.3 | 3 | 1 | 3 | 4 |
| Nr. 4.4 | 4 | 3 | 4 | 4 |
| Nr. 5.1 | 1 | 1 | 3 | 3 |
| Nr. 6.1 | 4 | 4 | 4 | 4 |

Boniturschema

0 = keine Wirkung
1 = schwache Schädigung
2 = mittlere Schädigung
3 = starke Schädigung
4 = total vernichtet

Species

Ec = Echinochloa crus-galli
Ce = Cyperus esculentus
Cd = Cyperus difformis
Fi = Fimbristylis miliacea

Anwendungsbeispiel E

Im Gewächshaus wurden die in der Tabelle aufgeführten Verbindungen mit den ebenfalls erwähnten Aufwandmengen appliziert. Hierzu wurden die Wirkstoffe in Gefäße mit 1500 ml Wasser gegeben (Wasserapplikation). Die Testpflanzen wurden im 2-bis 5-Blattstadium eingesetzt. Drei Wochen nach der Applikation wurde die Schädigung der Pflanzen bonitiert.

Wie die Tabelle zeigt, sind die erfindungsgemäßen Verbindungen stark wirksam gegen wichtige Reisunkräuter.

Wasserapplikation

| Verbindung | ppm | Ec | Ce | Cd | Fi | Sa | El |
|---|---|---|---|---|---|---|---|
| Nr. 3.2 | 10 | 4 | 1 | 3 | 3 | - | - |
| Nr. 4.3 | 10 | 4 | 1 | 4 | 4 | - | - |
| Nr. 4.4 | 1 | 4 | 3 | 4 | 4 | - | - |
| | 3 | 4 | 3 | 4 | 4 | - | - |
| Nr. 5.1 | 1 | 4 | 2 | 2 | 2 | - | - |
| | 3 | 4 | 4 | 3 | 4 | - | - |
| Nr. 6.1 | 1 | 4 | 4 | 4 | 4 | - | - |
| | 3 | 4 | 4 | 4 | 4 | - | - |
| Nr. 7.1 | 3 | 4 | - | 4 | - | 3 | 4 |
| Nr. 8.1 | 3 | 4 | - | 4 | - | 3 | 4 |
| Nr. 9.1 | 3 | 4 | - | 4 | - | 3 | 4 |

Boniturschema

0 = keine Wirkung
1 = schwache Schädigung
2 = mittlere Schädigung
3 = starke Schädigung
4 = total vernichtet
- = nicht geprüft

Species

Ec = Echinochloa crus-galli
Ce = Cyperus esculentus
Cd = Cyperus difformis
Fi = Fimbristylis miliacea
Sa = Saggitaria pusilla
El = Eleocharis acicularis

**Ansprüche**

1. Halogencyclopropyl-Verbindungen der allgemeinen Formel I

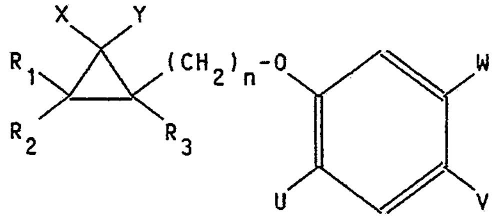

(I) ,

$R_1$, $R_2$ und $R_3$ unabhängig voneinander Wasserstoff oder $C_{1-4}$-Alkyl,
X Wasserstoff oder Halogen,
Y Halogen,
n 0, 1, 2 oder 3,
U und V Wasserstoff oder Halogen und
W einen heterocyclischen Rest der Formeln

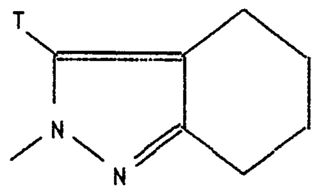 ,

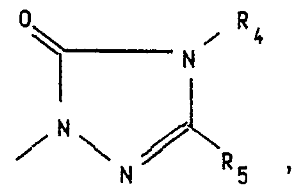 ,

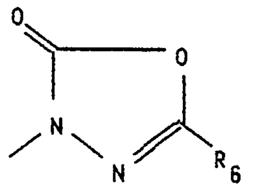 ,

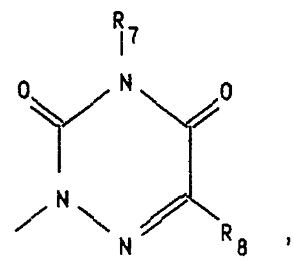 ,

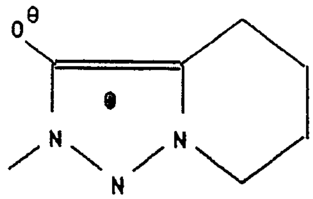 ,

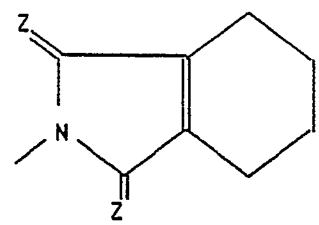

oder 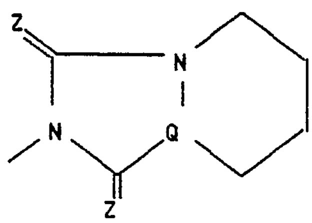

bedeuten, wobei
T für Halogen, $C_{1-4}$-Alkyl, $C_{1-4}$-Halogenalkyl, CN oder $OR_9$,
Q für CH oder N,
Z für O oder S,
$R_4$, $R_5$, $R_6$, $R_7$ und $R_8$ jeweils für einen geradkettigen, verzweigten oder cyclischen, gegebenenfalls mit bis zu sechs Halogenatomen substituierten $C_{1-7}$-Alkylrest stehen, wobei
$R_4$ und $R_5$ außerdem gemeinsam einen heterocyclischen 4-bis 7-gliedrigen Ring bilden können, der gesättigt oder ungesättigt sein kann, weitere Heteroatome wie O, S oder N enthalten kann und gegebenenfalls mit ein bis drei Methylgruppen oder ein bis sechs Halogenatomen substituiert sein kann, und
$R_9$ Wasserstoff, C $_{1-4}$-Alkyl oder $C_{1-4}$-Halogenalkyl bedeutet.

2. Verfahren zur Herstellung von Halogencyclopropyl-Verbindungen der Formel I, dadurch gekennzeichnet, daß man
a) ein Halogenid der allgemeinen Formel II

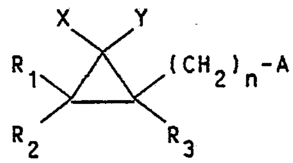

(II) ,

worin $R_1$, $R_2$, $R_3$, X, Y und n die in Formel I genannte Bedeutung haben und A Chlor, Brom oder Jod bedeutet, mit einem Phenol der Formel III

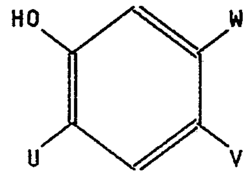

(III) ,

worin U, V und W die allgemeinen Formel I genannte Bedeutung haben, umsetzt,
oder
b) falls W einen 4,5,6,7-Tetrahydroindazol-2-yl-Rest darstellt, eine Verbindung der Formel IV

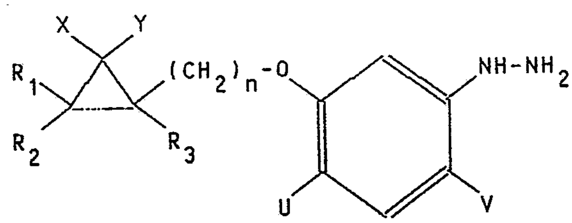

(IV) ,

worin $R_1$, $R_2$, $R_3$, X, Y, n, U und V die in Formel I genannte Bedeutung haben, mit einer Verbindung der Formel V

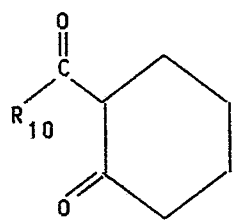

(V) ,

worin $R_{10}$ $C_{1-4}$-Alkyl, $C_{1-4}$-Halogenalkyl oder $C_{1-4}$ -Alkoxy bedeutet, umsetzt und gewünschtenfalls die so erhaltenen Verbindungen der Formel I mit T in der Bedeutung von Hydroxy -mit Dialkylsulfat in die entsprechenden Verbindungen mit T in der Bedeutung von Alkoxy oder mit Phosphoroxyhalogeniden in die entsprechenden Verbindungen mit T in der Bedeutung von Halogen und durch Umsetzung mit NaCN in die entsprechenden Verbindungen mit T in der Bedeutung von CN überführt,
oder
c) falls W einen 2H-1,2,4-Triazolin-3-on-2-yl-Rest darstellt eine Verbindung der Formel IV

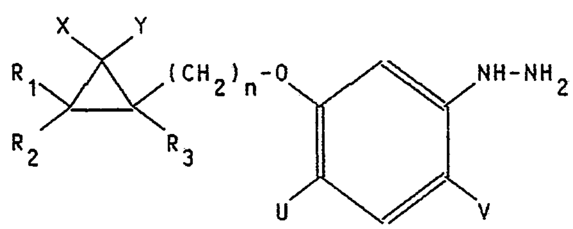

(IV) ,

worin $R_1$, $R_2$, $R_3$, X, Y, n, U und V die in Formel I genannte Bedeutung haben, mit einer Verbindung der

Formel VI

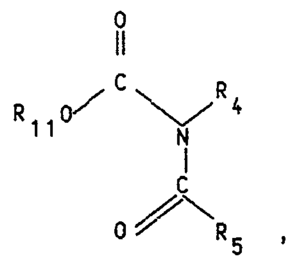

(VI) ,

worin $R_4$ und $R_5$ die in der Formel I genannte Bedeutung haben und $R_{11}$ $C_{1-4}$-Alkyl bedeutet, umsetzt, oder

d) falls W einen 3H-1,3,4-Oxadiazol-3-on-3-yl-Rest darstellt, eine Verbindung der Formel IV

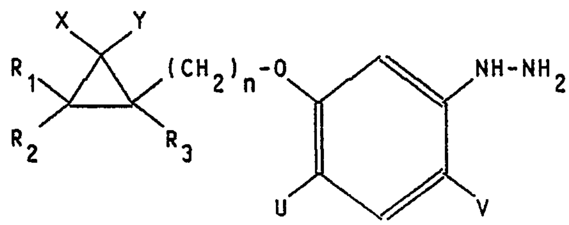

(IV) ,

worin $R_1$, $R_2$, $R_3$, X, Y, n, U und V die in Formel I genannte Bedeutung haben, mit einem Säurederivat der Formel VIII

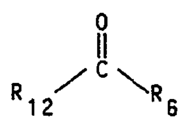

(VIII) ,

worin $R_6$ die in Formel I angegebene Bedeutung hat und $R_{12}$ Halogen oder $C_{1-4}$-Alkoxy bedeutet, intermediär zu einer Verbindung der Formel VII

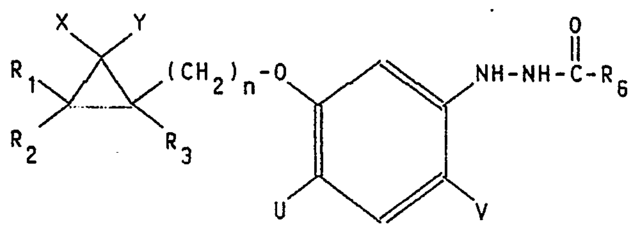

(VII) ,

der $R_1$, $R_2$, $R_3$, X, Y, n, U, V und $R_6$ die in der Formel I genannte Bedeutung haben, umsetzt und dann mit Phosgen oder einem seiner reaktiven funktionellen Derivate zur Reaktion bringt,
oder

e) falls W einen 2H, 4H-1,2,4-Triazin-3,5-dion-2-yl-Rest darstellt, eine Verbindung der Formel IV

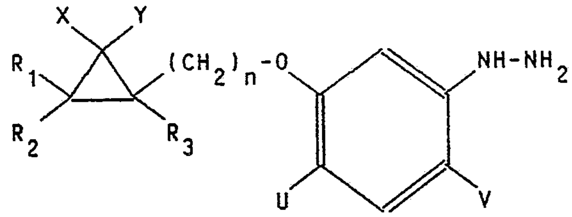

(IV) ,

worin $R_1$, $R_2$, $R_3$, X, Y, n, U und V die in Formel I genannte Bedeutung haben, mit einer Ketosäure der Formel XII

$R_8-\overset{O}{\overset{\|}{C}}-COOH$ (XII) ,

worin $R_8$ die in Formel I genannte Bedeutung hat, zu einer Verbindung der Formel XI

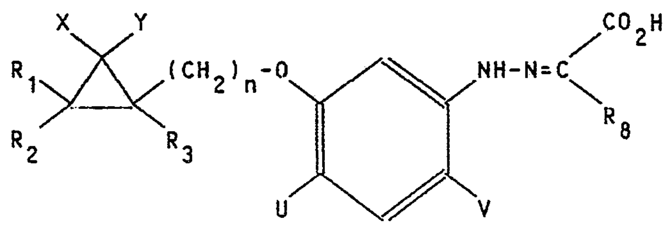

(XI) ,

worin $R_1$, $R_2$, $R_3$, X, Y, n, U, V und $R_8$ die in Formel I genannte Bedeutung haben, mit Thionylchlorid oder Phosphoroxychlorid umsetzt und anschließend mit Carbaminsäureestern cyclisiert und die so erhaltene Verbindung der Formel IX

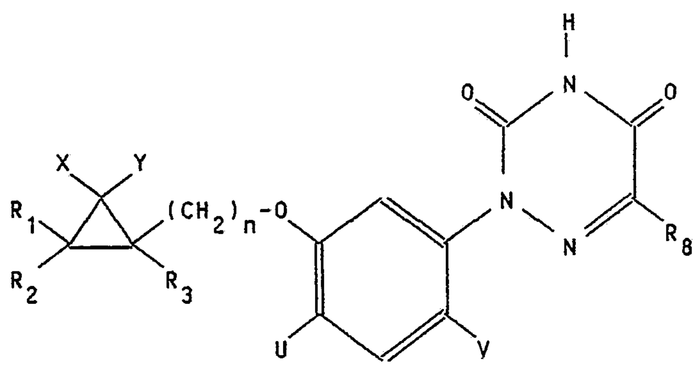

(IX) ,

worin $R_1$, $R_2$, $R_3$, X, Y, n, U, V und $R_8$ die in Formel I genannte Bedeutung haben, mit einer Verbindung der Formel X

$R_7$-A (X) ,

worin $R_7$ die in Formel I genannte Bedeutung hat und A Chlor, Brom oder Jod bedeutet, umsetzt,

oder

f) falls W einen 4,5,6,7-Tetrahydro-2H-1,2,3-triazolo-[3,4-a]pyridin-8-ium-3-olat-2-yl-Rest darstellt, eine Verbindung der Formel XV

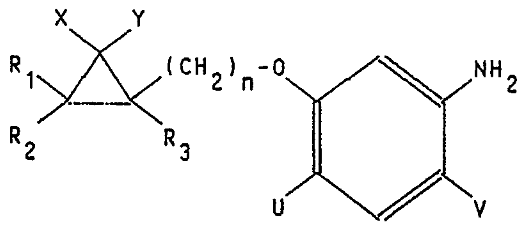

(XV) ,

worin $R_1$, $R_2$, $R_3$, X, Y, n, U und V die in Formel I genannte Bedeutung haben, mit salpetriger Säure diazotiert, mit Piperidin-2-carbonsäure umsetzt und mit Acetanhydrid cyclisiert,
oder
g) falls W einen 1,3,4,5,6,7-Hexahydro-2H-isoindol-1,3-di-on-2-yl-Rest darstellt, eine Verbindung der Formel XV

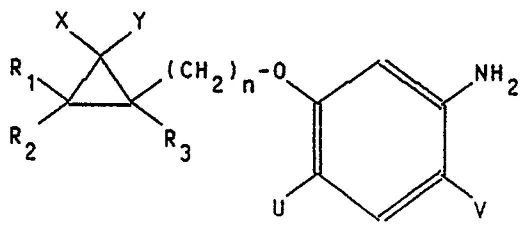

(XV) ,

worin $R_1$, $R_2$, $R_3$, X, Y, n, U und V die in Formel I genannte Bedeutung haben, mit einer Verbindung der Formel XVI

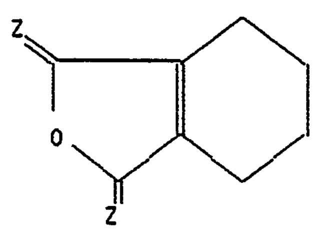

(XVI) ,

in der Z = O oder S bedeutet, umsetzt,
oder
h) falls W einen 2,3,5,6,7,8-Hexahydro-1H-imidazo[1,5-a]pyridin-1,3-dion-2-yl-oder einen 2,3,5,6,7,8-Hexahydro-1H-1,3,4-triazolo[1,2-a]pyridazin-1,3-dion-2-yl-Rest darstellt, eine Verbindung der Formel XV

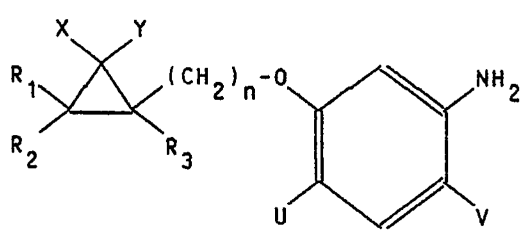

(XV) ,

worin $R_1$, $R_2$, $R_3$, X, Y, n, U und V die in Formel I genannte Bedeutung haben, mit einer Verbindung der Formel XVII oder XVIII

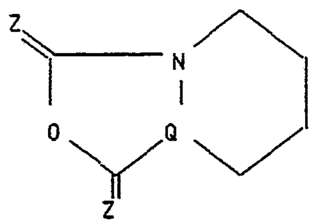 (XVII) 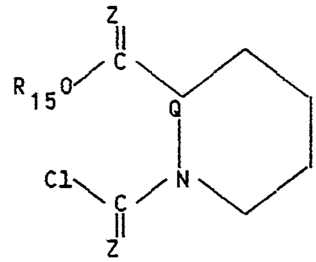 (XVIII),

in denen Z = O oder S, Q = CH oder N und $R_{15}$ $C_{1-4}$-Alkyl bedeuten, umsetzt
oder
ein Verbindung der Formel XV mit

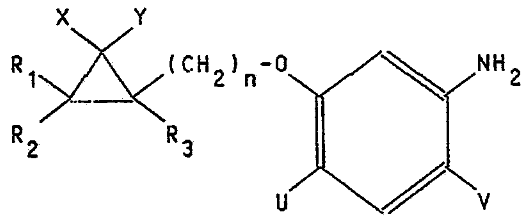

(XV) ,

worin $R_1$, $R_2$, $R_3$, X, Y, n, U und V die in Formel I genannte Bedeutung haben, mit Phosgen oder Thiophosgen intermediär zu einer Verbindung der Formel XIX

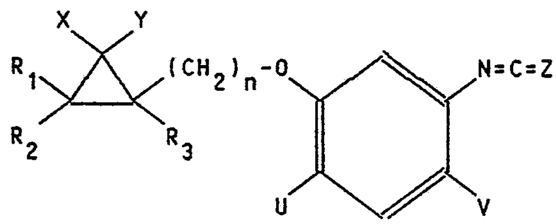

(XIX) ,

worin $R_1$, $R_2$, $R_3$, X, Y, n, U, V und Z die in Formel I genannte Bedeutung haben, umsetzt und anschließend mit einer Verbindung der Formel XX

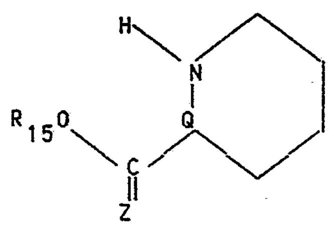

(XX) ,

worin Q = CH oder N, Z = O oder S und $R_{15}$ einen $C_{1-4}$-Alkylrest bedeuten, zur Reaktion bringt,
oder
i) eine Verbindung der Formel XXI

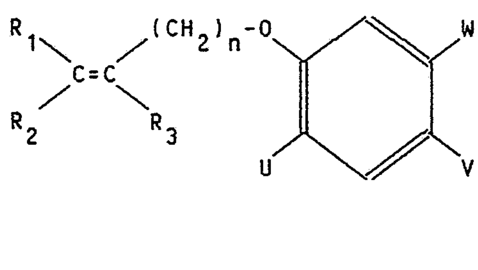

(XXI) ,

worin $R_1$, $R_2$, $R_3$, X, Y, n, U und V die in Formel I genannte Bedeutung haben, mit halogenierten Methanderivaten der Formel XXII

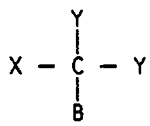

(XXII) ,

in der X und Y die in der Formel I genannte Bedeutung haben und B Wasserstoff oder

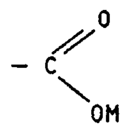

bedeutet, wobei M Alkalimetall darstellt, umsetzt.

3. Mittel mit herbizider Wirkung, gekennzeichnet durch einen Gehalt an mindestens einer Verbindung gemäß Anspruch 1.

4. Mittel gemäß Anspruch 3 in Mischung mit Träger-und/oder Hilfsstoffen.

5. Verwendung der Verbindungen gemäß Anspruch 1 zur Bekämpfung dikotyler und monokotyler Pflanzenarten in Nutzpflanzkulturen.